(19) Europäisches Patentamt

European Patent Office

Office européen des brevets

(11) **EP 1 338 288 B1**

(12) **EUROPEAN PATENT SPECIFICATION**

(45) Date of publication and mention
of the grant of the patent:
**31.05.2006 Bulletin 2006/22**

(51) Int Cl.:
*A61K 47/38* (2006.01)    *A61K 9/16* (2006.01)
*A61K 9/14* (2006.01)

(21) Application number: **01979030.2**

(22) Date of filing: **06.11.2001**

(86) International application number:
**PCT/JP2001/009691**

(87) International publication number:
**WO 2002/036168 (10.05.2002 Gazette 2002/19)**

(54) **CELLULOSIC PARTICLES FOR PHARMACEUTICAL PREPARATIONS**

ZELLULOSEPARTIKEL FÜR EINE PHARMAZEUTISCHE ZUSAMMENSETZUNG

PARTICULES CELLULOSIQUES DESTINEES A DES PREPARATIONS PHARMACEUTIQUES

(84) Designated Contracting States:
**AT BE CH CY DE DK ES FI FR GB GR IE IT LI LU MC NL PT SE TR**

(30) Priority: **06.11.2000 JP 2000338243**

(43) Date of publication of application:
**27.08.2003 Bulletin 2003/35**

(73) Proprietor: **Asahi Kasei Kabushiki Kaisha Osaka-shi,
Osaka 530-8205 (JP)**

(72) Inventors:
• **GOMI, Shun'ichi**
  **Nobeoka-shi, Miyazaki 882-0036 (JP)**
• **KAMADA, Etsuo**
  **Nobeoka-shi, Miyazaki 882-0003 (JP)**
• **HIRANO, Yuuji**
  **Nobeoka-shi, Miyazaki 882-0802 (JP)**

(74) Representative: **Blake, John Henry Francis et al
Brookes Batchellor LLP
102-108 Clerkenwell Road
London EC1M 5SA (GB)**

(56) References cited:
EP-A- 1 300 420          EP-A2- 0 452 862
JP-A- 3 111 426          US-A- 4 159 345
US-A- 5 505 983

**Description**

TECHNICAL FIELD

The present invention relates to cellulose particles for pharmaceutical use, a process for producing the same, a method of use thereof and active ingredient-containing granules produced by using these cellulose particles.

BACKGROUND ART

There are various processes for preparing effective ingredient-containing granules. In recent years, there has been proposed a process comprising layering an active ingredient on seed particles. In particular, as a pharmaceutical machine is improved, it becomes possible to layer a large amount of the active ingredients onto relatively small seed particles. As a result, there are proposed various processes for layering active ingredients.

For example, JP-A 61-1614 discloses a process comprising layering active ingredients on sugar-type seed cores; JP-A 7-173050 and JP-A 4-283520 disclose a process comprising layering active ingredients on a spherical seed cores, and JP-A 2000-109426 discloses a process comprising layering active ingredients on microcrystalline cellulose.

However, the sugar-type seed cores disclosed in JP-A 61-1614 have problems such that:

(1) it is difficult to prepare a seed core having a small particle size suitable for the layering of active ingredients,

(2) when the layering of active ingredients is carried out using said sugar-type seed cores and an aqueous active ingredients suspension, a sugar, which is a main component of the seed cores, itself dissolves making the surface sticky, and as a result, aggregation of the seed cores themselves easily occurs, and

(3) the seed cores are weak and are easily worn away due to abrasion in the course of fluidization, so that aggregations of the seed cores themselves and adhesion of the seed cores to the walls of a coating machine easily occur, and as a result, yield deteriorates.

Further, in the case where the spherical seed cores disclosed in JP-A 7-173050 or JP-A 4-283520 are used as the seed cores, such spherical seed cores have problems such that:

(1) although the spherical seed cores are hardly worn away due their resistance to abrasion and have superior flowability, it is necessary to increase supplied air flow in order to ensure the flowability required for the layering of active ingredients, and the layered active ingredients are easily stripped, depending upon the air flow in the course of fluidization or due to collision of seed cores amongst themselves, because of their weight,

(2) although the spherical seed cores are superior in their water absorption, aggregation easily occurs in an early stage of the layering when the spraying rate of an aqueous active ingredients suspension is increased, because its surface is flat, and

(3) in the case where it is applied for the preparation of intraoral rapidly soluble tablets, it feels rough to the tongue when administered, because the spherical seed core has too great a strength.

Furthermore, in the case where microcrystalline cellulose is used as the seed particle as disclosed in JP-A 2000-109426, there remains a problem in that it is difficult to layer active ingredients, because

(1) the particle has a low tapped bulk density, so that it adheres to any bag filter mounted to the upper portion of a coating machine, when air is supplied in a flow suitable for the layering of active ingredients,

(2) it has a large angle of repose, so that flowability in the coating machine becomes poor, and

(3) it is easily worn away due to abrasion, so that granule(s) having a narrow particle size range cannot be obtained.

US 5,505,938 discloses spherical pharmacologically inactive seed cores which comprise at least 50% by weight of microcrystalline cellulose and show the following characteristics:

- average degree of polymerization of 60-375
- average particle size of 100-1000 micrometers tapped bulk density of at least 0.65 g/ml
- aspect ratio of at least 0.7
- a water absorption capacity of 0.5-1.5 mg/g
- a friability of no more than 1%.

These cores are coated with a powder comprising a pharmacologically active ingredient.

US 4,159,345 discloses particles of an excipient which essentially consists of microcrystalline cellulose having an average degree of polymerization of 60-375 and a particle size of 20 or 35 micrometers.

EP 1 300 420 discloses cellulose powder having an average L/D value of particles of 75 micrometers. an average polymerization degree of 150-450 or 230-450, an average particle size of 20-250 micrometers, an apparent specific volume of 4-7 cm$^3$/g, an apparent tapping specific volume of 2.4-4.5 cm$^3$/g, an angle of repose of 55° or less.

DISCLOSURE OF INVENTION

The present inventors have undertaken extensive studies on properties of a particle used in the pharmaceutical field, and as a result, the present invention has been accomplished.

The present invention provides:

(1) cellulose particles for pharmaceuticals, which contain microcrystalline cellulose having an average degree of polymerization of from 60 to 350 in an amount of not less than 10%, and which have a tapped bulk density of from 0.60 to 0.95 g/ml, an aspect ratio not less than 0.7, a shape factor of from 1.10 to 1.50, and an average particle size of from 10 to 400 μm;

(2) a process for producing the cellulose particles according to the above item (1), which comprises the steps of hydrolyzing a cellulose material so as to obtain an average degree of polymerization of from 60 to 350, successively mechanically milling a thus hydrolyzed product so as to obtain an average particle size of not more than 15 μm, preparing a dispersion liquid containing the obtained microcrystalline cellulose, forming said dispersion liquid into droplets, and thereafter drying said droplets; and

(3) spherical granules characterized in that a medicine is contained on the surface or inside of the cellulose particles according to the above item (1).

BEST MODE FOR CARRYING OUT THE INVENTION

The cellulose particles for pharmaceuticals in accordance with the present invention contain microcrystalline cellulose having an average degree of polymerization of from 60 to 350 (hereinafter simply referred to as microcrystalline cellulose) in an amount of not less than 10%. When the microcrystalline cellulose is contained in an amount of not less than 10%, the particles can be given appropriate strength, and loss due to abrasion in the course of layering of active ingredients can be diminished. From the viewpoint of particle strength and resistance to abrasion, it is preferable that the cellulose particles for pharmaceuticals contain the microcrystalline cellulose in an amount of not less than 30%, preferably not less than 50%, and more preferably not less than 70%. The most preferred is 100% of the microcrystalline cellulose from the viewpoint of simplification of the pharmaceutical formulation. It is not recommended that the microcrystalline cellulose content be less than 10%, because the particle strength is low and the loss due to abrasion is large.

The microcrystalline cellulose to be used has an average degree of polymerization of from 60 to 350. It can be obtained by subjecting a cellulose material such as cotton linters, pulp or regenerated fiber to hydrolysis such as acid hydrolysis, alkali hydrolysis, steam explosion decomposition or a combination of two or three thereof. Alternatively, a mechanical treatment such as pulverization may be applied before or after the chemical treatment mentioned above.

It is not recommended that the average degree of polymerization exceeds 350, because a behavior of fiber is observed, so that it is difficult to mill the microcrystalline cellulose and moreover the aspect ratio is lowered. It is not recommended that the average degree of polymerization be less than 60, because entanglement of the cellulose molecule is diminished, so that hardness of the cellulose particle used for pharmaceuticals becomes insufficient. A preferable average degree of polymerization is from 100 to 270, and more preferable is from 120 to 200.

In the particle, ingredients other than the microcrystalline cellulose may be incorporated. Examples thereof are a binder agent (for example, hydroxypropyl cellulose, hydroxypropylmethyl cellulose, polyvinyl alcohol, polyvinylpyrrolidone, macrogol and the like), a film coating agent (for example, hydroxypropylmethyl cellulose phthalate, hydroxypropylmethyl cellulose acetate succinate, carboxymethylethyl cellulose, ethyl cellulose, ethyl cellulose aqueous dispersion, aminoalkyl methacrylate copolymer E, methacrylic acid copolymer L, methacrylic acid copolymer S, methacrylic acid copolymer LD, aminoalkyl methacrylate copolymer RS, hardened oil and the like), a surface active agent (for example, sucrose fatty acid ester, polyoxyethylene polyoxypropylene glycol, polysorbate, sodium laurylsulfate and the like), an excipient (for example, corn starch, potato starch, rice starch, powdered sugar, lactose, D-mannitol, trehalose, microcrystalline cellulose and carboxymethylcellulose sodium, and the like), a disintegrant (for example, low substitution hydroxypropyl cellulose, carboxymethylcellulose calcium, cross-carmellose sodium, pregelatinized starch and the like), an inorganic substance (for example, talc, magnesium stearate, light silicic acid anhydride, synthetic aluminum silicate, titanium oxide and the like), and other additives which can be conventionally used for the pharmaceuticals.

The cellulose particles for pharmaceuticals in accordance with the present invention have a tapped bulk density of from 0.60 to 0.95 g/ml. It is not recommended that the tapped bulk density is less than 0.60 g/ml, because such particles are so light in weight, that it is necessary to decrease the rate at which particles are fed into the layering machine.

Further, the reason why it is not recommended is because the particles adhere to any bag filter mounted to the upper portion of a coating machine under a condition of an air volume suitable for the layering of active ingredients, thereby resulting in deterioration of yield. While, it is not recommended that the tapped bulk density exceeds 0.95 g/ml, because the particles are so heavy in weight, that it is necessary to greatly increase air volume required for the fluidization of the particles suitable for the layering of active ingredients(s). As a result, the active ingredient(s) is easily stripped due to the high air flow rate, resulting in fluctuations in the layering of active ingredient(s). A preferable tapped bulk density is from 0.60 to 0.90 g/ml. More preferable is from 0.60 to 0.85 g/ml, and particularly preferable is from 0.65 to 0.85 g/ml.

The cellulose particles for pharmaceuticals in the present invention have an aspect ratio of not less than 0.70. When the aspect ratio is less than 0.7, the granules after the layering of active ingredient(s) deteriorate in their aspect ratio, and it is not desirable from the viewpoint of the aesthetic appearance of a product. Preferably, the aspect ratio is not less than 0.75, and more preferably it is not less than 0.80.

The cellulose particles for pharmaceuticals used in the present invention have a shape factor of from 1.10 to 1.50. It is not recommended that the shape factor be less than 1.10, because the surface of each particle is smooth and therefore either an aqueous active ingredient suspension or a binder solution is difficult to adhere. Increase of spraying rate is not recommended, because aggregation easily occurs in the course of the layering. On the other hand, it is not recommended that the shape factor exceeds 1.50, because the surfaces of the particles are too irregular, so that a lot of irregularity appears on granules after the layering of active ingredient(s), thereby resulting in poor aesthetic appearance. Moreover, the reason why it is not recommended is because the particles are easily worn away due to abrasion, thereby likely causing aggregation of the particles, and as a result, the granules have a broad and particle size ranges. Furthermore, it is not recommended that granules having a lot of irregularity be coated, because it is difficult to control release rate of the active ingredient(s). A preferable shape factor is from 1.15 to 1.50, and more preferable is from 1.15 to 1.45.

The cellulose particles for pharmaceuticals used in the present invention have an average particle size of from 10 to 400 $\mu$m. It is not recommended that the average particle size be less than 10 $\mu$m, because the layering of active ingredient(s) is difficult to carry out and aggregation of particles themselves easily occurs. Further, it is not recommended that the average particle size exceeds 400 $\mu$m, because there is produced layered granules having a large particle size, which likely feels rough to the tonque, thereby deteriorating dose facility. Moreover, such a large particle size results in fluctuation of content at the time of use in granule-containing tablets. Furthermore, it is not recommended because the amount of active ingredient(s) to be layered is limited in the case in which granules having a small particle size are required. The average particle size is preferably from 40 to 400 $\mu$m, more preferably from 50 to 400 $\mu$m, much more preferably from 50 to 300 $\mu$m, and particularly preferably from 50 to 200 $\mu$m.

It is preferable that the cellulose particles used for pharmaceuticals in accordance with the present invention have a specific surface area of from 0.15 to 0.60 m$^2$/g. It is not preferable that the specific surface area be less than 0.15 m$^2$/g wherein the surfaces of the particles are flat, so that either an aqueous active ingredient suspension or a binder solution becomes difficult to adhere. Increase of spraying rate is not preferable because aggregation can easily occur in the course of the layering. It is not preferable that the specific surface area exceeds 0.60 m$^2$/g, because the particles are easily worn away by abrasion, thereby likely causing aggregation of the particles, thus producing granules with a broad and uneven particle size ranges.

It is preferable that the cellulose particles for pharmaceuticals in accordance with the present invention have a water vapor absorption of not less than 1.50%. It is not preferable that the water vapor absorption be less than 1.50%, because the water-absorbing property is insufficient, likely resulting in aggregation of particles and adhering of the same to the walls of a coating machines when an aqueous active ingredient(s) suspension is sprayed or when an aqueous binder solution is used to layer the active ingredient(s).

It is preferable that the cellulose particles for pharmaceutical(s) in accordance with the present invention have a loading peak value of from 130 to 630 mN. It is not preferable that the loading peak value be less than 130 mN, because the particles have insufficient strength, and therefore the particle(s) are easily worn away due to abrasion or cracked during the course of the layering, thereby causing aggregation of the particles, thus producing granules with a broad and uneven particle size ranges. It is not preferable that the loading peak value exceeds 630 mN, because the particles have too great a strength, thereby resulting in feeling rough to the tongue when administered.

It is preferable that the cellulose particles for pharmaceuticals in accordance with the present invention have an angle of repose of not more than 41°. It is not preferable that the angle of repose exceeds 41°, because flowability deteriorates, thereby resulting in aggregation of the particles in the course of the layering of active ingredient. The angle of repose is more preferably not more than 39°, and much more preferably not more than 37°.

It is desired that the cellulose particles for pharmaceuticals are low in friability. It is not desired that the friability be high, because the particles are easily worn away by abrasion, thereby causing aggregation of the particles, thus producing granules having a broad and uneven particle size ranges.

The process for producing cellulose particles for pharmaceuticals in accordance with the present invention necessarily comprises a wet milling step and a spray-drying step as mentioned below. When the process comprising these two steps is applied, it is possible to obtain cellulose particles for pharmaceuticals, which have physical properties as defined herein. It is otherwise very difficult to obtain the cellulose particles for pharmaceuticals in accordance with the present invention, particularly cellulose particles satisfactory in both a relatively small average particle size and a large shape factor.

For example, the cellulose particles for pharmaceuticals in accordance with the present invention can be produced by the following process.

First of all, a cellulose material such as cotton linters, pulp or regenerated fiber is subjected to hydrolysis such as acid hydrolysis, alkali hydrolysis, steam explosion decomposition or a combination of two or three thereof, so as to obtain a degree of polymerization of from 60 to 350. Before the hydrolysis, a mechanical treatment as mentioned below may be applied. A dispersion of cellulose itself obtained through filtration, washing or decantation of the resulting hydrolysis mixture has a conductivity of preferably not more than 300 $\mu$S/cm. It is not preferable that the conductivity exceeds 300 $\mu$S/cm, because impurities produced at the time of hydrolysis contaminate the cellulose particles for pharmaceuticals. The dispersion is purified so as to obtain a conductivity of preferably not more than 150 $\mu$S/cm, and particularly preferably not more than 75 $\mu$S/cm, thereby obtaining a cake-like product, which is then subjected to the successive wet milling step.

In the wet milling step, the cake-like product obtained above is mechanically treated by means of, for example, a milling treatment. Or it is converted into a dispersion containing the cake-like product, followed by mechanical treatment by means of, for example, a milling treatment. Alternatively, it is treated by means of a combination thereof. Thereby, the particle size of microcrystalline cellulose in the cellulose dispersion is not more than 15 $\mu$m. It is not recommended that the particle size exceeds 15 $\mu$m, because the tapped bulk density of the cellulose particles for pharmaceuticals becomes low and particle strength decreases. The particle size is preferably not more than 13 $\mu$m, and more preferably not more than 10 $\mu$m.

When the purification is carried out by means of decantation or the like, the raw cellulose dispersion may be subjected to mechanical treatment.

Alternatively, microcrystalline cellulose powder alone, obtained by drying the cellulose dispersion may be subjected to pulverization or milling treatment as mentioned above and thereafter mixed with water, thereby obtaining the desired particle size. Or the powder may be mixed with water to obtain a suspension, which is then subjected to milling treatment as mentioned above, thereby obtaining the desired particle size. Alternatively, the powder may be treated according to a combination of the above-mentioned procedures. Further, the powder obtained by pulverizing or milling microcrystalline cellulose, once dried, may be combined with the microcrystalline cellulose whose particle size has been adjusted through wet milling after hydrolysis, thereby obtaining the cellulose dispersion.

The mechanical treatment can be carried out in a conventional manner. One embodiment is as follows. When the cake-like product is to be treated, the pulverization can be carried out at a solids content of, for example, from 25 to 80%, preferably from 30 to 60% using a blend-stirring machine (for example, an all-purpose blend-stirring machine or the like) or a knead-milling machine (for example, a mortar, a kneader or the like). When the dispersion is to be treated, the pulverization can be carried out at a solids content of from 1 to 30% using a blend-dispersing machine (for example, a homogenizer, a high-pressure homogenizer or the like), or a medium milling machine (for example, a wet vibration mill, a wet planetary vibration mill, a wet ball mill, a wet roll mill, a wet beads mill, a wet paint shaker or the like).

The resulting milled cake-like product and the milled cellulose-containing dispersion are diluted to a concentration of about 1 to 25%, thereby obtaining a dispersion of cellulose itself, whose particle diameter can be easily controlled to a desired degree in the succeeding drying step mentioned below. The dispersion of cellulose itself is adjusted to pH 5 to 8.5.

When ingredients other than the microcrystalline cellulose are incorporated therein, said ingredients may be blended with the cake-like product after hydrolysis of cellulose, or may be blended with the cellulose dispersion obtained according to the above-mentioned procedures.

Successively, the foregoing cellulose dispersion is converted to droplets and then dried by means of spray drying with the use of a rotary disk, a two-flow paired nozzle, a pressure nozzle or the like.

A machine to be used for the spray drying and a method of the spray drying are not limited. However, a spray drying method with use of a rotary disk is recommended from the viewpoint such that characteristics of the cellulose particles for pharmaceuticals in accordance with the present invention can be imparted. In carrying out the spray drying method using a rotary disk, the liquid feeding rate, the solids content of the liquid, the diameter of the rotary disk, the speed of the rotary disk and the drying temperature are not particularly limited. One embodiment is as follows. A microcrystalline cellulose-containing dispersion having a solids content of 1% or more is supplied to a rotary disk having a diameter of from 3 to 50 cm operated at a rotary disk speed of from 500 to 30000 rpm, while controlling the liquid feeding rate and the drying temperature, thereby obtaining the desired particle size.

The solids content in the microcrystalline cellulose dispersion, subjected to the spray drying, is preferably from 1 to 25%. It is preferable that the solids content is 1% or more, because it is difficult to obtain cellulose particles for pharmaceuticals having a desired average particle size due to insufficient aggregation of the particles. Also from the viewpoint of drying efficiency, it is preferable. On the other hand, it is not preferable that the solid content exceeds 25%, because the cellulose dispersion increases in its viscosity, and therefore coarse particles are likely to be produced after drying. The solids content is more preferably from 3 to 20%, and most preferably from 5 to 20%.

The cellulose particles for pharmaceuticals after drying have a loss on drying of preferably not more than 10%, more preferably not more than 7%, and particularly preferably not more than 5%.

After completion of the spray drying, if desired, sieving and classification may be carried out, thereby limiting the average particle size to 10 to 400 $\mu$m. From the viewpoint of properties of the cellulose particles for pharmaceuticals, it is preferable that the particle size ranges be narrow.

The cellulose particles for pharmaceuticals in accordance with the present invention are obtained by drying a dispersion containing microcrystalline cellulose milled to the extent of the desired particle size into aggregate droplets. Therefore, they are high in aspect ratio, high in tapped bulk density, appropriate in shape factor, easily regulated in particle size ranges, appropriate in specific surface area, large in water vapor absorption and appropriate in particle strength.

In order to layer active ingredient on the cellulose particles for pharmaceuticals, a conventional method can be used. There are mentioned the following methods for layering active ingredient, which are not intended to limit the mode for carrying out the present invention.

That is, (1) a method comprising spraying a liquid prepared by dissolving or suspending active ingredients in a solution of a binder agent, while fluidizing the cellulose particles for pharmaceuticals with a fluidized bed granulation coating apparatus (or a rotating fluidized bed type granulation coating apparatus, a fluidized bed granulation coating apparatus equipped with a Wurster column or a fluidized bed granulation coating apparatus equipped with an improved Wurster column), (2) a method comprising continuously spraying a solution of a binder agent and at the same time feeding powder of active ingredient (and an excipient, if necessary), while rolling the cellulose particles for pharmaceuticals in a centrifugal fluidized type coating apparatus, (3) a method comprising adding active ingredients in an amount capable of being absorbed by the particle and binder solution, while rolling the cellulose particles for pharmaceuticals with a high speed mixing granulation apparatus, and (4) a method comprising immersing the cellulose particles for pharmaceuticals in active ingredient and binder solution. In any method, operations such as removal of dried and aggregated particles may be carried out when required.

The active ingredient used in the present invention is that used for treatment, prophylaxis or diagnosis of human or animal diseases.

Examples thereof are antiepileptic drugs (acetylpheneturide, primidone and the like), antipyretic, analgesic and anti-inflammatory drugs (acetaminophen, phenyl acetylglycine dimethylamide, diclofenac sodium, oxyphenbutazone, sulpyrine, ibuprofen, ketoprofen, tinolidine hydrochloride, benzydamine hydrochloride, tiaramide hydrochloride, piroxicam and the like), anti-vertigenous drugs (dimenhydrinate, difenidol hydrochloride and the like), psychoneurosis drugs (chloropromazine hydrochloride, levomepromazine maleate, perazine maleate, perphenazine, diazepam, oxazepam and the like), skeletar muscle relaxants (chlorzoxazone, chlorphenesin carbamate, chlormezanone, eperisone hydrochloride and the like), autonomic nervous system drugs (bethanechol chloride, neostigmine bromide, pyridostigmine bromide and the like), antispasmodic drugs (butropium bromide, scopolamine-N-butylbromide, propantheline bromide, papaverine hydrochloride and the like), antiparkinson drugs (trihexyphenidyl hydrochloride and the like), antihistamine drugs (diphenhydramine hydrochloride, dl-chlorpheniramine maleate, d-chlorpheniramine maleate, promethazine, mequitazine and the like), cardiotonic drugs (aminophylline, caffeine, dl-isoproterenol hydrochloride, etilefrine hydrochloride and the like), antiarrhythmic drugs (disopyramide and the like), diuretic drugs (potassium chloride, hydrochlorothiazide, acetazolamide and the like), blood pressure descendent drugs (hexamethonium bromide, hydralazine hydrochloride, propranolol hydrochloride, captopril, methyldopa and the like), vasodilator drugs (etafenone hydrochloride, carbocromen hydrochloride, pentaerythritol tetranitrate, dipyridamole, nicametate citrate and the like), arteriosclerosis drugs (lecithin and the like), circulatory organs drugs (nicardipine hydrochloride, meclofenoxate hydrochloride, pyridinol carbamate, calcium hopantenate, pentoxifylline and the like), respiratory acceleration drugs (dimefline hydrochloride and the like), antitussive-expectorant drugs (dextromethorphan hydrobromide, noscapine, L-methylcysteine hydrochloride, theophylline, ephedrine hydrochloride and the like), cholagogue drugs (dehydrocholic acid and the like), digestive organs drugs (metoclopramide, domperidone and the like), vitamins (fursultiamine, octotiamine, pyridoxine hydrochloride, nicotinic acid, ascorbic acid and the like), anti-biotic substances (erythromycin, kitasamycin, josamycin, tetracycline and the like), and chemotherapy drugs (isoniazid, ethionamide, nitrofurantoin, enoxacin, ofloxacin, norfloxacin and the like). In the present invention, it is permitted to layer two or more active ingredients simultaneously or one after another.

The amount of the active ingredients to be layered is determined by the dose. Incidentally, the amount referred to here means an amount of the active ingredients to be layered on the surface of the cellulose particles for pharmaceuticals. One embodiment elaborately given is as follows. The amount to be layered is about 0.01% by weight based on the weight of an original granule when the effect of the active ingredients can be expected even at an extremely low dose. It is about 500% by weight when a large dose is required to achieve active ingredient effect.

In layering an active ingredient(s) on the cellulose particles for pharmaceuticals, the active ingredient(s) may be layered together with additives for the purpose of facilitating each operation, preventing the active ingredient(s) from stripping off at a post-processing step, controlling the dissolution rate of the active ingredient(s) or enhancing stabilization. Examples of additives are: a binder agent (for example, hydroxypropyl cellulose, hydroxypropylmethyl cellulose, polyvinyl alcohol, polyvinylpyrrolidone, macrogol and the like), a film coating agent (for example, hydroxypropylmethyl cellulose phthalate, hydroxypropylmethyl cellulose acetate succinate, carboxymethylethyl cellulose, ethyl cellulose, ethyl cellulose aqueous dispersion, aminoalkyl methacrylate copolymer E, methacrylic acid copolymer L, methacrylic acid copolymer S, methacrylic acid copolymer LD, aminoalkyl methacrylate copolymer RS, hardened oil and the like), a surface active agent (for example, sucrose fatty acid ester, polyoxyethylene polyoxypropylene glycol, polysorbate, sodium laurylsulfate and the like), an excipient (for example, corn starch, rice starch, powdered sugar, lactose, microcrystalline cellulose, powdered cellulose, microcrystalline cellulose and carboxymethylcellulose sodium and the like), a disintegrant (for example, low-substitution hydroxypropyl cellulose, carmellose calcium, cross carmellose sodium, pregelatinized starch and the like), an inorganic substance (for example, talc, magnesium stearate, light silicic acid anhydride, synthetic aluminum silicate, titanium oxide and the like), and others.

The medium used in the layering of an active ingredient(s) is not limited. Water, ethanol and other organic solvents usable for pharmaceuticals may be used. A liquid prepared by suspending or dissolving the active ingredeint (s) and the binder agent in the solvent may be used for the layering.

One of preferred modes is as follows. For the purpose of improving ease of taking, improving appearances, excluding moisture, excluding oxygen, regulating the dissolution rate of the active ingredient (for example, preparing a controlled release drug or an enteric drug) or masking bitterness or odor of active ingredient(s), an aqueous film-coating or a solvent film-coating is applied to granules obtained by layering the active ingredient(s) on the cellulose particles in accordance with the present invention.

A film-coating agent used for such a film-coating may be a conventional one. Examples thereof are a water soluble one (for example, aminoalkyl methacrylate copolymer E, hydroxypropyl cellulose, hydroxypropylmethyl cellulose and the like), a release-controlled one (for example, ethyl cellulose, ethyl cellulose aqueous dispersion, methacrylic acid copolymer S, aminoalkyl methacrylate copolymer RS, ethyl acrylate-methyl methacrylate copolymer emulsion and the like), and an enteric one (for example, hydroxypropylmethyl cellulose phthalate, hydroxypropylmethyl cellulose acetate succinate, carboxymethylethyl cellulose, methacrylic acid copolymer L, methacrylic acid copolymer LD, methacrylic acid copolymer S and the like). Further, the coating can be applied using the above-mentioned coating agent alone or a combination of two or more thereof, if desired, together with other water soluble materials for the purposes of performing bitterness masking, odor masking, moisture proofing and oxygen proofing. However, the film-coating agent is not limited only thereto.

For coating the film comprising the foregoing film-coating agent, a conventional means may be used. There are mentioned a fluidized bed granulation coating apparatus, a fluidized bed granulation coating apparatus equipped with a Wurster column or a fluidized bed granulation coating apparatus equipped with an improved Wurster column, a centrifugal fluidized bed type granulation coating apparatus and a rotating fluidized bed type granulation coating apparatus.

The layered active ingredient-carrying granules in accordance with the present invention or the film-coated granules obtained by applying film-coating to the layered active ingredient-carrying granule(s) for the purpose of controlling a dissolution rate or the like may be administered dosed as is. Or they may be used after encapsulation or in a combination with other medicines. Alternatively, they may be mixed with the other excipient, active ingredient(s), active ingredient-containing granules or film-coated granules, and then formed into a tablet to be used in the form of a granules-containing tablet.

Methods for measuring physical properties of the cellulose particles for pharmaceuticals are summarized as follows:

• Degree of polymerization of microcrystalline cellulose

The microcrystalline cellulose confirmation test (3) set forth in Pharmacopoeia Japonica, the 13th Ed. was used.

• Particle size of milled particle [$\mu$m]

After an aqueous cellulose dispersion before drying was diluted with water to obtain a concentration showing a suitable transmittance and sonicated for one minute, an average particle size by volume was measured using a laser diffraction type size distribution analyzer (Type LA-910, manufactured by Horiba, Ltd.) under conditions of a relative refractive index of 1.2 and an uptaking frequency of 10, while stirring the diluted dispersion.

• pH

An aqueous cellulose dispersion before drying was adjusted to 25°C, and a pH thereof was measured using a glass electrode type hydrogen ion densitometer (Type D-21, manufactured by Horiba, Ltd.).

• Conductivity (IC) [$\mu$S/cm]

An aqueous cellulose dispersion before drying was adjusted to 25°C, and a conductivity thereof was measured using a conductivity measuring apparatus (Type D-21, manufactured by Horiba, Ltd.).

• Tapped bulk density of cellulose particles for pharmaceuticals [g/ml]

Thirty grams of particles were roughly filled into a 100 ml glass measuring cylinder, and hand-tapping was carried out on a low shock support such as a desk covered with a rubber sheet. The tapping was carried out to vertically drop the cylinder from a height of several cm onto the support until the particles were no longer compressed. After completion of the tapping, the volume of the particle layer was measured and divided by 30. The measurement was repeated three times, and the results were averaged.

• Aspect ratio of cellulose particle for pharmaceuticals

Images taken by a digital microscope (Type VH-7000 with VH-501 lens, manufactured by KEYENCE Co.) were stored in the form of TIFF file, 1360 x 1024 pixels, and using an image processing analysis software (Image Hyper II, developed by DegiMo Co.), 100 particles were processed to obtain their aspect ratios of cross section (shorter size/longer size), which were then averaged.

• Shape factor of cellulose particle for pharmaceuticals

Images taken by a digital microscope (Type VH-7000 with VH-501 lens, manufactured by KEYENCE Co.) were stored in the form of TIFF file, 1360 x 1024 pixels, and using an image processing analysis software, 100 particles were processed to obtain their shape factors, which were then averaged. The shape factor can be obtained using the following equation. When a sphere has no irregularity, the value is 1. The irregularity increases with increase in the value, namely a value larger than 1.

$$\text{Shape factor} = (\text{perimeter of particles})^2 / (4\pi \times (\text{sum of the areas of particles}))$$

• Average size of cellulose particles for pharmaceuticals [$\mu$m]

Using a Ro-Tap sieve shaker (Sieve Shaker Type A, manufactured by Hirako Seisaku-sho Co., Ltd.), the sample (30 g) was screened using a JIS standard sieve (Z8801-1987) for 15 minutes, thereby measuring particle ranges. The grain size of 50% by weight accumulated was taken as an average particle size. The measurement was repeated three times, and the results were averaged.

In the case where the cellulose particles for pharmaceuticals has an average particle size of less than 38 $\mu$m, the particles are dispersed in water. Thereafter, the average particle size was measured according to the above-mentioned method for measuring the size of the milled particles.

Specific surface area of cellulose particles for pharmaceuticals

The sample was dried for 3 hours at 105°C in a blast thermostatic drier (Type FC-610, manufactured by Toyo Seisakusho Kaisha, Ltd.) and fed to a measurement cell so as to keep a passage for a gas. The cell was mounted to a de-gas portion of a flow specific surface area automatic measuring apparatus (Flowsorb 2300, manufactured by Shimadzu Corporation), and degassing was carried out for 15 minutes at 120°C using a mantle heater. After removal of water vapor attached to the inside wall of the cell, a specific surface area was measured at a flow rate of nitrogen gas/(nitrogen gas + helium gas) of 0.3. The measurement was repeated three times, and the results were averaged.

• Water vapor absorption of cellulose particles for pharmaceuticals

The sample was dried for 3 hours at 105°C in a blast thermostatic drier. Thereafter, the sample (about 30 mg) was put in a dynamic vapor adsorption measuring apparatus (Type DVS-1, manufactured by Surface Measurement Systems Ltd.), and dried under fixed conditions of a temperature of 25°C, atmosphere of nitrogen gas and a relative humidity (RH) of 0% until the particle weight sufficiently reached equilibrium (weight fluctuation was not higher than 0.02%). Thereafter, the relative humidity was fixed to 5% RH and it was allowed to stand up to equilibrium (weight fluctuation was not higher than 0.02%). Successively, the relative humidity was fixed at 10% and it was allowed to stand up to equilibrium (weight fluctuation was not higher than 0.02%). Thereafter, the procedure was repeated with a change in the relative humidity by 5% at a time, namely 15%RH, 20%RH, 25%RH, 30%RH and 35%RH, thereby finding a difference between the particle weight at a relative humidity of 30% and that at a relative humidity of 0%. A ratio (%) of the difference weight to the particle weight at a relative humidity of 0% is defined as water vapor absorption. The measurement was repeated three times, and the results were averaged.

• Loading peak value of cellulose particles for pharmaceuticals

These valves were measured at a measuring speed of 250 μm/sec with use of a granule strength measuring apparatus (GRANO, manufactured by Okada Seiko Co., Ltd.), provided that an inflection point of inclination in a waveform of relative displacement and loading was taken as the loading peak value. In the displacement of 50% or less of the particle diameter of measured particles, the point at which no change of loading was observed over a displacement width two or more times that of the minimum moving step (1 μm) in a top chip of the granule strength measuring apparatus, or the first point from which the loading decreased over the same displacement width was taken as the inflection point. One hundred particles whose inflection points could be detected were averaged.

• Repose angle of cellulose particles for pharmaceuticals (°)

Measurement was carried out using a powder tester (Type PT-R, manufactured by Hosokawamicron Co.) and repeated three times. The results thereof were averaged.

The present invention is explained in detail with reference to the following Examples.

Example 1

Commercially available Kraft pulp (hereinafter simply referred to as KP) was cut into chips, and hydrolyzed in an aqueous 10% hydrochloric acid solution at 105°C for 30 minutes. The resulting acid insoluble residue was filtered and washed to obtain a cake-like product of microcrystalline cellulose having a solids concentration of about 40%. The cake-like product was found to have a degree of polymerization of 153. As shown in Table 1, the degree of polymerization was the same as that of particles obtained through milling and drying of the cake-like product. The cake-like product was milled for 1 hour with an all-purpose blend-stirring machine (Type 5DM-03-R, manufactured by Sanei Seisakujo Co.). Water was added thereto. Using a homo mixer (T.K. HOMO MIXER MARK 2II, manufactured by TokushuKika Kogyo Co., Ltd.), the mixture of the milled cake-like product and water was formed into a cellulose dispersion having a solids content of 12.5% by weight. After regulating the particle size, pH and IC, the dispersion was spray-dried using an about 8 cm rotary disk under conditions of a disk rotating speed of about 5000 rpm, a flow rate of about 6 l/hr, a supplied air temperature of about 170°C and an exhaust temperature of about 85°C. Coarse particles were removed using a sieve having a mesh of 177 μm, and fine particles were removed by passing through a sieve having a mesh of 75 μm. As a result, cellulose particles for pharmaceuticals A were obtained. The particle size of milled particles in the cellulose dispersion before drying and the physical properties of the cellulose particles for pharmaceuticals A are as shown in Table 1.

Example 2

Water was added to the cake-like product milled in Example 1, and the mixture was formed into a cellulose dispersion having a solids content of 15% by weight with a homogenizing mixer. After regulating the particle size, pH and IC, the dispersion was spray-dried under the same conditions as in Example 1. Coarse particles were removed using a sieve having a mesh of 212 μm, and fine particles were removed by passing through a sieve having a mesh of 75 μm. As a result, cellulose particles for pharmaceuticals B were obtained. The particle size of milled particles in the cellulose dispersion before drying and the physical properties of the cellulose particles for pharmaceuticals B are as shown in Table 1.

Example 3

Water was added to the cake-like product having a solids content of about 40% obtained in Example 1, and the mixture was formed into a cellulose dispersion having a solids content of 10% by weight with a homogenizing mixer. The dispersion was passed three times through a high pressure crushing apparatus (MICROFLUIDIZER Type M-610, manufactured by Microfluidics Co.) under a pressure of 120 MPa, thereby completing the crushing treatment. After regulating the particle size, pH and IC, the dispersion was spray-dried under the same conditions as in Example 1, except that the supplied air temperature was changed to 180°C. Coarse particles were removed using a sieve having a mesh of 75 $\mu$m, and fine particles were removed using a sieve having a mesh of 45 $\mu$m. As a result, cellulose particles for pharmaceuticals C were obtained. The particle size of milled particles in the cellulose dispersion before drying and the physical properties of the cellulose particles for pharmaceuticals C are as shown in Table 1.

Example 4

Water was added to the cake-like product milled in Example 1, and the mixture was formed into a cellulose dispersion having a solids content of 18% by weight with a homogenizing mixer. After regulating the particle size, pH and IC, the dispersion was spray-dried under the same conditions as in Example 1. Coarse particles were removed using a sieve having a mesh of 212 $\mu$m, and fine particles were removed by passing through a sieve having a mesh of 106 $\mu$m. As a result, cellulose particles for pharmaceuticals D were obtained. The particle size of milled particles in the cellulose dispersion before drying and the physical properties of the cellulose particles for pharmaceuticals D are as shown in Table 1.

Example 5

Commercially available Dissolving pulp (hereinafter simply referred to as DP) was cut into chips, and hydrolyzed in an aqueous 10% hydrochloric acid solution at 105°C for 10 minutes. The resulting acid insoluble residue was filtered and washed to obtain a cake-like product having a solids concentration of about 40%. The cake-like product was milled for 1 hour with an all-purpose blend-stirring machine. Water was added thereto. Using a homogenizing mixer, the mixture of the milled cake-like product and water was formed into a cellulose dispersion having a solids content of 15% by weight. The dispersion was passed three times through a high pressure crushing apparatus under a pressure of 120 MPa, thereby completing a crushing treatment. After regulating the particle size, pH and IC, the dispersion was spray-dried under the same conditions as in Example 1, except that the rotary disk rotating speed was changed to 4000 rpm. Coarse particles were removed using a sieve having a mesh of 300 $\mu$m, and fine particles were removed using a sieve having a mesh of 177 $\mu$m. As a result, cellulose particles for pharmaceutials E were obtained. The particle size of milled particles in the cellulose dispersion before drying and the physical properties of the cellulose particles for pharmaceuticals E are as shown in Table 1.

Example 6

Commercially available KP was cut into chips, and hydrolyzed in an aqueous 10% hydrochloric acid solution at 105°C for 60 minutes. The resulting acid insoluble residue was filtered and washed to obtain a cake-like product having a solids concentration of about 40%. The cake-like product was milled for 1 hour with an all-purpose blend-stirring machine. Water was added thereto. Using a homogenizing mixer, the mixture of the milled cake-like product and water was formed into a cellulose dispersion having a solids content of 13% by weight. The dispersion was passed three times through a high pressure crushing apparatus under a pressure of 120 MPa, thereby completing the crushing treatment. After regulating the particle size, pH and IC, the dispersion was spray-dried under the same conditions as in Example 1. Coarse particles were removed using a sieve having a mesh of 150 $\mu$m, and fine particles were removed by passing through a sieve having a mesh of 63 $\mu$m. As a result, cellulose particles for pharmaceuticals F were obtained. The particle size of milled particles in the cellulose dispersion before drying and the physical properties of the cellulose particles for pharmaceuticals F are as shown in Table 1.

Example 7

Commercially available DP was cut into chips, and hydrolyzed in an aqueous 3% hydrochloric acid solution at 105°C for 15 minutes. The resulting acid insoluble residue was filtered and washed to obtain a cake-like product having a solids concentration of about 40%. The cake-like product was milled for 1 hour with an all-purpose blend-stirring machine. Water was added thereto. Using a homogenizing mixer, the mixture of the milled cake-like product and water was formed into a cellulose dispersion having a solids content of 15% by weight. After regulating the particle size, pH and IC, the dispersion was spray-dried under the same conditions as in Example 1. Coarse particles were removed using a sieve having a mesh of 300 $\mu$m, and fine particles were removed by passing through a sieve having a mesh of 75 $\mu$m. As a result, cellulose particles for pharmaceuticals G were obtained. The particle size of milled particles in the cellulose dispersion before drying and the physical properties of the cellulose particles for pharmaceuticals G are as shown in Table 2.

Example 8

The cake-like product obtained in Example 7 was milled for 30 minutes with an all-purpose blend-stirring machine. Water was added thereto. Using a homogenizing mixer, the mixture of the milled cake-like product and water was formed into a cellulose dispersion having a solids content of 20% by weight. After regulating the particle size, pH and IC, the dispersion was spray-dried under the same conditions as in Example 1, except that the flow rate was changed to about 6.5 l/hr and the rotary disk rotating speed was changed to 2000 rpm. Coarse particles were removed using a sieve having a mesh of 420 $\mu$m, and fine particles were removed by passing through a sieve having a mesh of 350 $\mu$m. As a result, cellulose particles for pharmaceuticals H were obtained. The particle size of milled particles in the cellulose dispersion before drying and the physical properties of the cellulose particles for pharmaceuticals H are as shown in Table 2.

Example 9

Water was added to the cake-like product milled in Example 1, and the mixture was formed into a cellulose dispersion having a solids content of 14% by weight with a homogenizing mixer. After regulating the particle size, pH and IC, the dispersion was mixed with an aqueous 12% by weight lactose solution in the proportion of 1000 g of the former to 500 g of the latter, and the mixture was spray-dried under the same conditions as in Example 1. Coarse particles were removed using a sieve having a mesh of 212 $\mu$m, and fine particles were removed by passing through a sieve having a mesh of 75 $\mu$m. As a result, cellulose particles for pharmaceuticals I were obtained. The particle size of milled particles in the cellulose dispersion before addition of the aqueous lactose solution and the physical properties of the cellulose particles for pharmaceuticals I are as shown in Table 2.

Example 10

Water was added to the cake-like product milled in Example 1, and the mixture was formed into a cellulose dispersion having a solids content of 9% by weight with a homogenizing mixer. After regulating the particle size, pH and IC, the dispersion was mixed with an aqueous 9% by weight lactose solution in the proportion of 1000 g of the former to 1000 g of the latter, and the mixture was spray-dried under the same conditions as in Example 1, except that the supplied air temperature was changed to about 180°C. Coarse particles were removed using a sieve having a mesh of 150 $\mu$m, and fine particles were removed by passing through a sieve having a mesh of 63 $\mu$m. As a result, cellulose particles for pharmaceuticals J were obtained. The particle size of milled particles in the cellulose dispersion before addition of the aqueous lactose solution and the physical properties of the cellulose particles for pharmaceuticals J are as shown in Table 2.

Example 11

Water was added to the cake-like product milled in Example 1, and the mixture was formed into a cellulose dispersion having a solids content of 8% by weight with a homogenizing mixer. After regulating the particle size, pH and IC, the dispersion was mixed with an aqueous 17.1% by weight lactose solution and an aqueous 3% by weight hydroxypropyl cellulose solution (Type L, manufactured by Nippon Soda Co., Ltd.) in the proportion of 500 g of the dispersion to 900 g of the lactose solution and 200 g of the hydroxypropyl cellulose solution, and the mixture was spray-dried under the same conditions as in Example 1, except that the supplied air temperature was changed to about 180°C. Coarse particles were removed using a sieve having a mesh of 150 $\mu$m, and fine particles were removed by passing through a sieve having a mesh of 75 $\mu$m. As a result, cellulose particles for pharmaceuticals K were obtained. The particle size of milled particles in the cellulose dispersion before addition of the aqueous lactose solution and the physical properties of the cellulose particles for pharmaceuticals K are as shown in Table 2.

Example 12

Water was added to the cake-like product milled in Example 1, and the mixture was formed into a cellulose dispersion having a solids content of 14.7% by weight with a homogenizing mixer. After regulating the particle size, pH and IC, the dispersion was mixed with an aqueous 1.5% by weight hydroxypropyl cellulose solution (Type L, manufactured by Nippon Soda Co., Ltd.) in the proportion of 1000 g of the former to 200 g of the latter, and the mixture was spray-dried under the same conditions as in Example 1. Coarse particles were removed using a sieve having a mesh of 300

μm, and fine particles were removed by passing through a sieve having a mesh of 150 μm. As a result, cellulose particles for pharmaceuticals L were obtained. The particle size of milled particles in the cellulose dispersion before addition of the aqueous hydroxypropyl cellulose solution and the physical properties of the cellulose particles for pharmaceuticals J are as shown in Table 2.

Example 13

The cake-like product having a solid content of about 40% obtained in Example 1 was milled for 90 minutes with an all-purpose blending machine. Water was added to the cake-like product milled, and the mixture was formed into a cellulose dispersion having a solid content of 3% by weight with a homogenizing mixer. After regulating the particle size, pH and IC, the dispersion was spray-dried using the same rotary disk as in Example 1 under the conditions of a rotary disk rotating speed of about 25000 rpm, a flow rate of about 3 l/hr, a supplied air temperature of about 180°C and an exhaust temperature of about 85°C. Coarse particles were removed using a sieve having a mesh of 38 μm, thereby obtaining cellulose particles for pharmaceuticals M. The cellulose particles for pharmaceuticals M were found to have a tapped bulk density of 0.62 g/ml. The particle size of milled particles in the cellulose dispersion and the physical properties of the cellulose particles for pharmaceuticals M are as shown in Table 3.

Example 14

The cake-like product obtained in Example 6 was milled for 1 hour with an all-purpose blend-stirring machine. Water was added to the cake-like product milled, and the mixture was formed into a cellulose dispersion having a solids content of 15% by weight with a homogenizing mixer. The dispersion was passed three times through a high pressure crushing apparatus under a pressure of 120 MPa, thereby completing the crushing treatment. After regulating the particle size, pH and IC, the dispersion was spray-dried under the same conditions as in Example 1, except that the rotary disk rotating speed was changed to 4000 rpm. Coarse particles were removed using a sieve having a mesh of 212 μm, and fine particles were removed using a sieve having a mesh of 75 μm. As a result, cellulose particles for pharmaceuticals N were obtained. The cellulose particles for pharmaceuticals N were found to have a tapped bulk density of 0.62 g/ml. The particle size of milled particles in the cellulose dispersion and the physical properties of the cellulose particles for pharmaceuticals N are as shown in Table 3.

Example 15

Water was added to the cake-like product milled in Example 1, and the mixture was formed into a cellulose dispersion having a solids content of 10.0% by weight with a homogenizing mixer. After regulating the particle size, pH and IC, the dispersion was mixed with an aqueous 10.0% by weight lactose solution, a 10.0% by weight corn starch dispersion and 5.0% by weight hydroxypropyl cellulose (Type L, manufactured by Nippon Soda Co., Ltd.) in the proportion of 300 g of the dispersion to 200 g of the lactose solution, 480 g of the corn starch dispersion and 40 g of the hydroxypropyl cellulose, and the mixture was spray-dried under the same conditions as in Example 1, except that the supplied air temperature was changed to 180°C. Coarse particles were removed using a sieve having a mesh of 150 μm, and fine particles were removed by passing through a sieve having a mesh of 75 μm. As a result, cellulose particles for pharmaceuticals O were obtained. The particle size of milled particles in the cellulose dispersion before addition of the aqueous lactose solution and the physical properties of the cellulose particles for pharmaceuticals O are as shown in Table 3.

Comparative Example 1

Water was added to the cake-like product having a solid content of about 40% obtained in Example 1, and the mixture was formed into a cellulose dispersion having a solids content of 15% by weight with a homogenizing mixer. After regulating the particle size, pH and IC, the dispersion was spray-dried under the same conditions as in Example 1. Coarse particles were removed using a sieve having a mesh of 212 μm, and fine particles were removed by passing through a sieve having a mesh of 45 μm. As a result, particles P were obtained. The particle size of the cellulose particles in the cellulose dispersion before drying and the physical properties of the particles P are as shown in Table 3.

Comparative Example 2

Commercially available DP was cut into chips, and hydrolyzed in an aqueous 1% hydrochloric acid solution at 105°C for 10 minutes. The resulting acid insoluble residue was filtered and washed to obtain a cake-like product having a solid concentration of about 40%. The cake-like product was milled for 10 minutes with an all-purpose blend-stirring machine. Water was added thereto. Using a homogenizing mixer, the mixture of the milled cake-like product and water was formed into a cellulose dispersion having a solids content of 15% by weight. After regulating the particle size, pH and IC, the dispersion was spray-dried under the same conditions as in Example 1. Coarse particles were removed using a sieve having a mesh of 300 $\mu$m, and fine particles were removed by passing through a sieve having a mesh of 106 $\mu$m. As a result, particles Q were obtained. The particle size of milled particles in the cellulose dispersion before drying and the physical properties of the particles Q are as shown in Table 3.

Comparative Example 3

The cake-like product obtained in Comparative Example 2 was milled for 1 hour with an all-purpose blend-stirring machine. Water was added thereto. Using a homogenizing mixer, the mixture of the milled cake-like product and water was formed into a cellulose dispersion having a solids content of 10% by weight. The dispersion was passed three times through a high pressure crushing apparatus under a pressure of 120 MPa, thereby completing the crushing treatment. After regulating the particle size, pH and IC, the dispersion was spray-dried under the same conditions as in Example 1, except that the supplied air temperature was changed to 180°C. Coarse particles were removed using a sieve having a mesh of 250 $\mu$m, and fine particles were removed by passing through a sieve having a mesh of 75 $\mu$m. As a result, particles R were obtained. The particle size of milled particles in the cellulose dispersion before drying and the physical properties of the particles R are as shown in Table 3.

Comparative Example 4

According to the process disclosed in JP-A 7-173050, 1.5 kg of microcrystalline cellulose was put in a high speed stirring granulation machine (Type FM-VG-10, manufactured by Powrex Co.), 1.0 kg of water was added thereto, and the mixture was kneaded for 5 minutes. One kilogram of the obtained wet granules were transferred to a Malmerizer (Type Q-230, manufactured by Fuji Paudal Co., Ltd.), which was then rolled for 10 minutes at 500 rpm, while spraying water at a rate of 10 g/min, thereby forming the granule into spheres. Thereafter, the spheres were dried at 40°C night and day in a hot air drying machine (Type PV-211, manufactured by Tabai ESPEC Co.). Coarse particles were removed using a sieve having a mesh of 150 $\mu$m and fine particles were removed using a sieve having a mesh of 75 $\mu$m, thereby obtaining particles S. Physical properties of the particles S are as shown in Table 4.

Comparative Example 5

Preparation of particles was carried out in the same manner as in Comparative Example 4. Coarse particles were removed using a sieve having a mesh of 212 $\mu$m and fine particles were removed using a sieve having a mesh of 106 $\mu$m, thereby obtaining particles T. Physical properties of the particles T are as shown in Table 4.

Comparative Example 6

Preparation of particles was carried out in the same manner as in Comparative Example 4, except that water to be added was increased up to 1.2 kg. Coarse particles were removed using a sieve having a mesh of 300 $\mu$m and fine particles were removed using a sieve having a mesh of 212 $\mu$m, thereby obtaining particles U. Physical properties of the particles U are as shown in Table 4.

Comparative Example 7

Commercially available crystalline cellulose (AVICEL (registered trademark) PH-200, manufactured by FMC Co.) was passed through a sieve having a mesh of 45 $\mu$m to remove fine particles, thereby obtaining particles V. Physical properties of the particles V are as shown in Table 4.

Comparative Example 8

Rayon scrap yarn was cut into chips, and hydrolyzed in an aqueous 10% hydrochloric acid solution at 100°C for 40 minutes. The resulting acid insoluble residue was washed with hot water according to decantation, and then formed into a cellulose dispersion having a solids content of 10% by weight. After regulating the particle size, pH and IC, the dispersion was spray-dried under the same conditions as in Example 1, except that a supplied air temperature was changed to 180°C. Coarse particles were removed using a sieve having a mesh of 75 $\mu$m, and fine particles were removed using a sieve having a mesh of 45 $\mu$m, thereby obtaining particles W. The particle size of milled particles in the cellulose dispersion before drying and the physical properties of the particles W are as shown in Table 4.

Comparative Example 8

A commercially available glucide spherical seed core (NONPAREIL (registered trademark) NP101 "32-42" (purified sugar : corn starch = 7 : 3), manufactured by Freund Industrial Co., Ltd.) was passed through a sieve having a mesh of 420 μm to remove coarse particles, thereby obtaining particles X. Physical properties of the particles X are as shown in Table 4.

Example 16

Using a rotating fluidized bed type granulation coating apparatus ("MULTIPLEX" Type MP-01, manufactured by Powrex Co.) with a tangential bottom spray, 1.0 kg of the cellulose particles for pharmaceuticals A obtained in Example 1 were fluidized for 20 minutes under the following conditions: rotating speed of rotary plate: 450 rpm, sprayed air pressure: 0.16 MPa, sprayed air flow rate: 40 l/min, protective air pressure: 0.20 MPa, supplied air temperature: room temperature (no heater), exhaust temperature: room temperature, and air volume: 40 $m^3$/hr. Results relating to flowability of the particle, friability thereof and adhesion to bag filter(s) thereof are as shown in Table 5.

Example 17

The particles B obtained in Example 2 were fluidized for 20 minutes under the same conditions as in Example 16. Results relating to flowability of the particle, friability thereof and adhesion to bag filter(s) thereof are as shown in Table 5.

Comparative Example 10

The particles P obtained in Comparative Example 1 were fluidized for 20 minutes under the same conditions as in Example 14. Results relating to flowability of the particle, friability thereof and adhesion to bag filter(s) thereof are as shown in Table 5.

Comparative Example 11

The particles V obtained in Comparative Example 7 were fluidized for 20 minutes under the same conditions as in Example 16. Results relating to flowability of the particle, friability thereof and adhesion to bag filter(s) thereof are as shown in Table 5.

Example 18

Under the same conditions as in Example 16, except that an air delivery was changed to 50 $m^3$/hr, 1.0 kg of the cellulose particles for formulation H obtained in Example 8 were fluidized for 10 minutes. Results relating to flowability of the particle, friability thereof and adhesion to bag filter(s) thereof are as shown in Table 6.

Comparative Example 12

Under the same conditions as in Example 18, the particles X obtained in Comparative Example 9 were fluidized for 10 minutes. Results relating to flowability of the particle, friability thereof and adhesion to bag filter(s) thereof are as shown in Table 6.

Example 19

Using Type MP-01 with an improved Wurster column, 1.0 kg of the cellulose particles for pharmaceuticals C obtained in Example 3 were fluidized for 20 minutes under conditions of sprayed air pressure: 0.16 MPa, sprayed air flow rate: 40 l/min, side air pressure: 0.20 MPa, side air delivery time: 0.2 sec, side air interruption time: 3.0 sec, supplied air temperature: room temperature (no heater), exhaust temperature: room temperature, and air volume: 30 $m^3$/hr. Results relating to flowability of the particle, friability thereof and adhesion to bag filter(s) thereof are as shown in Table 7.

Comparative Example 13

Under the same conditions as in Example 19, 1.0 kg of the particles W obtained in Comparative Example 8 were fluidized for 20 minutes. Results relating to flowability of the particle, friability thereof and adhesion to bag filter(s) thereof are as shown in Table 7.

Example 20

Under the same conditions as in Example 19, 1.0 kg of the cellulose particles for pharmaceuticals M obtained in Example 13 were fluidized for 20 minutes. Results relating to flowability of the particle, friability thereof and adhesion to bag filter(s) thereof are as shown in Table 8.

Comparative Example 14

Under the same conditions as in Example 20, 1.0 kg of the particles P obtained in Comparative Example 1, from which a fraction had been removed by passing through a sieve having a mesh of 38 $\mu$m, were fluidized for 20 minutes. Results relating to flowability of the particle, friability thereof and adhesion to bag filter(s) thereof are as shown in Table 8.

Example 21

Using Type MP-01 with a tangential bottom spray, an active ingredient solution containing 3 parts of caffeine, 2 parts of hydroxypropyl cellulose (Type L, manufactured by Nippon Soda Co., Ltd.) and 95 parts of water was sprayed in the proportion of 5.5 g/min to 0.5 kg of the cellulose particles for pharmaceuticals A obtained in Example 1 under conditions of rotating speed of rotary plate: 450 rpm, sprayed air pressure: 0.16 MPa, sprayed air flow rate: 40 l/min, protective air pressure: 0.20 MPa, supplied air temperature: 75°C, exhaust temperature: 35°C, and air volume: 30 m$^3$/hr, until the amount of caffeine layered on the cellulose particles for pharmaceuticals reached 2% by weight. The obtained granules were passed through a sieve having a mesh of 177 $\mu$m, and the proportion of the fraction as coarse particles to the granules was calculated. As shown in Table 9, it was confirmed that layering of the active ingredients could be carried out with almost no aggregation.

Comparative Example 15

Under the same conditions as in Example 21, caffeine was layered on 0.5 kg of the particles S obtained in Comparative Example 4. As shown in Table 9, it was found that aggregation more easily occurred as compared with the cellulose particles for pharmaceuticals A in Example 1.

Comparative Example 16

Using Type MP-01 with a tangential bottom spray, an active ingredient solution similar to that in Example 21 was sprayed in the proportion of 4.5 g/min to 0.5 kg of the particles V obtained in Comparative Example 7, from which coarse particles had been removed by passing through a sieve having a mesh of 177 $\mu$m and fine particles had been removed by passing through a sieve having a mesh of 75 $\mu$m, under conditions of rotating speed of rotary plate: 280 rpm, sprayed air pressure: 0.13 MPa, sprayed air flow rate: 30 l/min, protective air pressure: 0.10 MPa, supplied air temperature: 75°C, exhaust temperature: 36°C, and air volume: 30 m$^3$/hr, until the amount of caffeine layered on the particles reached 2% by weight. As shown in Table 9, it was found that aggregation more easily occurred as compared with the cellulose particles for pharmaceuticals A in Example 1.

Example 22

Under the same conditions as in Example 21, caffeine was layered on 0.5 kg of the cellulose particles B obtained in Example 2, until the amount of caffeine layered on the cellulose particles reached 2% by weight. The obtained granules were passed through a sieve having a mesh of 212 $\mu$m, and a proportion of the fraction as coarse particles to the granules was calculated. As shown in Table 10, it was confirmed that layering of the active ingredients could be carried out with almost no aggregation.

Comparative Example 17

Under the same conditions as in Example 22, caffeine was layered on 0.5 kg of the particles T obtained in Comparative Example 5, until the amount of caffeine layered on the particles reached 2% by weight. As shown in Table 10, it was found that aggregation more easily occurred as compared with the cellulose particles for pharmaceuticals B in Example 2.

Example 23

Using Type MP-01 with a tangential bottom spray, an active ingredients solution containing 4 parts of caffeine, 5 parts of hydroxypropyl cellulose (Type SL, manufactured by Nippon Soda Co., Ltd.) and 91 parts of water was sprayed in the proportion of 5.5 g/min to 0.8 kg of the cellulose particles for pharmaceuticals A obtained in Example 1 under the following conditions: rotating speed of rotary plate: 450 rpm, sprayed air pressure: 0.20 MPa, sprayed air flow rate: 40 l/min, protective air pressure: 0.20 MPa, supplied air temperature: 80°C, exhaust temperature: 37°C, and air volume: 30 $m^3$/hr, until the amount of caffeine layered on the cellulose particles for pharmaceuticals use reached 10% by weight. The obtained granules were passed through a sieve having a mesh of 212 $\mu$m, and a proportion of the fraction as coarse particles to the granules was calculated. As shown in Table 11, it was confirmed that layering of the active ingredients could be carried out with only a little aggregation.

Comparative Example 18

Under the same conditions as in Example 23, caffeine was layered on 0.8 kg of the particles S obtained in Comparative Example 4. As shown in Table 11, it was found that aggregation more easily occurred as compared with the cellulose particles for pharmaceuticals A in Example 1.

Example 24

Under the same conditions as in Example 23, caffeine was layered on 0.8 kg of the cellulose particles B obtained in Example 2 until the amount of caffeine layered on the cellulose particles reached 10% by weight. The obtained granules were passed through a sieve having a mesh of 250 $\mu$m, and a proportion of the fraction as coarse particles to the granules was calculated. As shown in Table 12, it was confirmed that layering of the active ingredients could be carried out with almost no aggregation.

Comparative Example 19

Under the same conditions as in Example 23, caffeine was layered on 0.8 kg of the particles T obtained in Comparative Example 5 until the amount of caffeine layered on the particles used reached 10% by weight. As shown in Table 12, it was found that aggregation more easily occurred as compared with the cellulose particles for pharmaceuticals B in Example 2.

Example 25

Using Type MP-01 with a tangential bottom spray, an active ingredients solution containing 4 parts of caffeine, 5 parts of hydroxypropyl cellulose (Type SL, manufactured by Nippon Soda Co., Ltd.) and 91 parts of water was sprayed in the proportion of 9.0 g/min to 1.0 kg of the cellulose particles for pharmaceuticals G obtained in Example 7, from which fine particles had been removed by passing through a sieve having a mesh of 212 $\mu$m, under the following conditions: rotating speed of rotary plate: 450 rpm, sprayed air pressure: 0.20 MPa, sprayed air flow rate: 40 l/min, protective air pressure: 0.20 MPa, supplied air temperature: 80°C, exhaust temperature: 36°C, and air volume: 40 $m^3$/hr, until the amount of caffeine layered on the cellulose particles for pharmaceuticals reached 10% by weight. The obtained granules were passed through a sieve having a mesh of 350 $\mu$m, and a proportion of the fraction as coarse particles to the granules was calculated. As shown in Table 13, it was confirmed that layering of the active ingredients could be carried out with only a little aggregation.

Comparative Example 20

Under the same conditions as in Example 25, caffeine was layered on 1.0 kg of the particles U obtained in Comparative Example 6. As shown in Table 13, it was found that aggregation more easily occurred as compared with the cellulose particles for pharmaceuticals use G in Example 7.

Example 26

Using Type MP-01 with an improved Wurster column, an active ingredients dispersion containing 10 parts of riboflavin, 2 parts of hydroxypropyl cellulose (Type SL, manufactured by Nippon Soda Co., Ltd.) and 88 parts of water was sprayed in a proportion of 5.0 g/min to 0.8 kg of the cellulose particles for pharmaceuticals M obtained in Example 13, under conditions of sprayed air pressure: 0.20 MPa, sprayed air flow rate: 40 l/min, side air pressure: 0.20 MPa, side air delivery time: 0.2 sec, side air interruption time: 3.0 sec, supplied air temperature: 75°C, exhaust temperature: 37°C, and air volume: 35 m$^3$/hr, until the amount of riboflavin layered on the cellulose particles for pharmaceuticals reached 2% by weight. The obtained granules were passed through a sieve having a mesh of 38 $\mu$m, and a proportion of the fraction as coarse particles to the granules was calculated. As shown in Table 14, it was confirmed that layering of the active ingredient could be carried out with only a little aggregation.

Comparative Example 21

Using Type MP-01 with an improved Wurster column, riboflavin was layered on 0.8 kg of the particles P obtained in Comparative Example 1, which had been passed through a sieve having a mesh of 38 m to remove a fraction, under the same conditions as in Example 21 except that sprayed air pressure, sprayed air flow rate and side air pressure were changed to 0.16 MPa, 30 l/min and 0.18 MPa, respectively. As shown in Table 14, it was found that aggregation more easily occurred as compared with the cellulose particles for pharmaceuticals M in Example 13.

Example 27

The layering of active ingredients was carried out twice under the same conditions as in Example 21, and the obtained granules were passed through a sieve having a mesh of 177 $\mu$m to remove a fraction. Using Type MP-01 with a tangential bottom spray, a coating liquid containing 38.1 parts of an aqueous ethyl cellulose dispersion, 2.9 parts of triacetin, 5.7 parts of D-mannitol and 53.3 parts of water (solid weight ratio/aqueous ethyl cellulose dispersion: triacetin: D-mannitol = 1.0: 0.25: 0.50) was sprayed to 0.7 kg of the above-prepared granules, under the following conditions: rotating speed of rotary plate: 450 rpm, sprayed air pressure: 0.18 MPa, sprayed air flow rate: 40 1/min, protective air pressure: 0.20 MPa, supplied air temperature: 70°C, exhaust temperature: 36°C, air volume: 40 m$^3$/hr, and coating liquid supplying speed: 7 g/min, until the amount of solids coated on the granules reached 25.0% by weight. The resulting coated granules were passed through a sieve having a mesh of 212 $\mu$m, and a proportion of the fraction as coarse particles to the coated granules was calculated. As shown in Table 15, it was confirmed that a coated granule could be obtained with only a little aggregation. The resulting granules were heat-treated at 80°C for 1 hour using a hot air drier, thereby completing film formation, and thereafter subjected to bitterness sensory analysis by 10 panelists. As shown in Table 15, there was found no sense of bitterness even after 30 seconds, thus confirming that bitterness masking could be performed.

Comparative Example 22

The lamination of active ingredients was carried out twice under the same conditions as in Comparative Example 16. The obtained granules were passed through a sieve having a mesh of 177 $\mu$m to remove a fraction and further passed through a sieve having a mesh of 75 $\mu$m to remove pulverized fine particles. Using Type MP-01 with a tangential bottom spray, the same coating liquid as in Example 18 was sprayed to 0.7 kg of the above-prepared granules, under the following conditions: rotating speed of rotary plate: 320 rpm, sprayed air pressure: 0.13 MPa, sprayed air flow rate: 30 l/min, protective air pressure: 0.10 MPa, supplied air temperature: 70°C, exhaust temperature: 37°C, air volume: 40 m$^3$/hr, and coating liquid supplying speed: 6 g/min, until the amount of solids coated on the granules reached 25.0% by weight. The resulting coated granules were passed through a sieve having a mesh of 212 $\mu$m to remove a fraction and further passed through a sieve having a mesh of 75 $\mu$m to remove pulverized fine particles. The proportion of the coarse particles to coated granules was calculated. As shown in Table 15, it was found that aggregation more easily occurred as compared with the granules obtained in Example 27. The same heat film formation treatment as in Example 27 was applied thereto. The resulting granules were subjected to the same bitterness sensory analysis as in Example 27. As shown in Table 15, there was found a sense of bitterness within an elapsed time of 30 seconds, thus confirming that bitterness masking could not be performed.

Example 28

50 parts of the coated granules obtained in Example 27, 17 parts of D-mannitol, 30 parts of microcrystalline cellulose (Ceolus KG-802, manufactured by Asahi Chemical Industry Co., Ltd.), and 3 parts of cross carmellose sodium were mixed for 3 minutes in a plastic bag, and 0.5 g of the sample was put in a mortar, and compressed with a flat pestle having a bottom area of 1 cm$^2$, in a compression machine (TESTSTAND MODEL-1321DW-CREEP, manufactured by AIKOH Engineering Co., Ltd.) at a compression rate of 1 cm/min, until the pressure reached 10 MPa, thereby obtaining a granule-containing tablet. The tablet was subjected to bitterness sensory analysis by 10 panelists. Dissolution in mouth was good, and there was found almost no sense of bitterness even after 30 seconds. As a result, it was confirmed that bitterness masking could be performed.

Comparative Example 23

A granule-containing tablet was prepared in the same manner as in Example 28, except that 50 parts of the coated granules obtained in Comparative Example 22 were used. The obtained tablet was subjected to the same sensory analysis as in Example 28. As a result, although dissolution in mouth was good, there was found a sense of bitterness immediately after dosage, thus confirming that bitterness masking could not be performed.

Example 29

Using Type MP-01 with a tangential bottom spray, an active ingredient dispersion containing 10 parts of riboflavin, 2 parts of hydroxypropyl cellulose (Type SL, manufactured by Nippon Soda Co., Ltd.) and 88 parts of water was sprayed in the proportion of 5.0 g/min to 1.0 kg of the cellulose particles for pharmaceuticals A obtained in Example 1, under the following conditions: rotating speed of rotary plate: 450 rpm, sprayed air pressure: 0.16 MPa, sprayed air flow rate: 40 l/min, protective air pressure: 0.20 MPa, supplied air temperature: 75°C, and exhaust temperature: 35°C, until the amount of riboflavin layered on the cellulose particles for pharmaceuticals reached 2% by weight. The obtained granules were passed through a sieve having a mesh of 177 $\mu$m to remove a fraction. Successively, using Type MP-01 with a tangential bottom spray, a coating liquid containing 38.1 parts of an aqueous ethyl cellulose dispersion, 2.9 parts of triethyl citrate, 1.4 parts of hydroxypropylmethyl cellulose (TC-5E, manufactured by Shin-Etsu Chemical Co., Ltd.) and 59.6 parts of water (solid weight ratio/aqueous ethyl cellulose dispersion: triethyl citrate: TC-5E = 1.0: 0.25: 0.125) was sprayed to 0.7 kg of the above-prepared granules, under the following conditions: rotating speed of rotary plate: 450 rpm, sprayed air pressure: 0.18 MPa, sprayed air flow rate: 40 l/min, protective air pressure: 0.20 MPa, supplied air temperature: 70°C, exhaust temperature: 36°C, air volume: 40 m$^3$/hr, and coating liquid supplying speed: 7 g/min, until the amount of solid coated on the granules reached 20.0% by weight. The coated granules were passed through a sieve having a mesh of 212 $\mu$m to remove coarse particles. The resulting granules were heat-treated at 80°C for 1 hour using a hot air drier, thereby completing film formation, and then subjected to a test according to the 2nd dissolution material test method in Pharmacopoeia Japonica, 13th Ed. (test liquid: 900 ml of a 1st liquid in a disintegration test method of a general test method in Pharmacopoeia Japonica, 13th Ed., paddle rotating speed: 100 rpm, with automatic dissolution test machine Type DT-610, manufactured by JASCO Co.). The average riboflavin dissolution ratio after an elapsed time of 4 hours, measured according to absorptiometry, was found to be 39% based on three runs.

Comparative Example 24

Using Type MP-01 with a tangential bottom spray, the same layered active ingredients dispersion as in Example 24 was sprayed in a proportion of 5 g/min to 1.0 kg of the particles V obtained in Comparative Example 7, which had been passed through a sieve having a mesh of 177 $\mu$m to remove coarse particles and further passed through a sieve having a mesh of 75 $\mu$m to remove fine particles, under the following conditions: rotating speed of rotary plate: 280 rpm, sprayed air pressure: 0.13 MPa, sprayed air flow rate: 30 l/min, protective air pressure: 0.10 MPa, supplied air temperature: 75°C, exhaust temperature: 36°C, and air volume: 30 m$^3$/hr, until the amount of riboflavin layered on the cellulose particles for pharmaceuticals reached 2% by weight. The obtained granules were passed through a sieve having a mesh of 177 $\mu$m to remove a fraction, and further passed through a sieve having a mesh of 75 $\mu$m to remove pulverized fine particles. Successively, using Type MP-01 with a tangential bottom spray, the same coating liquid as in Example 29 was sprayed to 0.7 kg of the above-prepared granules, under the following conditions: rotating speed of rotary plate: 320 rpm, sprayed air pressure: 0.13 MPa, sprayed air flow rate: 30 l/min, protective air pressure: 0.10 MPa, supplied air temperature: 70°C, exhaust temperature: 37°C, air volume: 40 m$^3$/hr, and coating liquid supplying speed: 5.5 g/min, until the amount of solids coated on the granules reached 20.0% by weight. The coated granules were passed through a sieve having a mesh of 212 $\mu$m to remove coarse particles, and further passed through a sieve having a mesh of 75 $\mu$m to remove pulverized fine particles. After carrying out the same heat treatment for film formation as in Example 24, the dissolution material test was carried out. As a result, a riboflavin dissolution ratio after an elapsed time of 4 hours was found to average 82% based on three runs. It was found that dissolution could not be controlled as compared with the coated granules in Example 29, notwithstanding the fact that the coating amount was the same in each case.

Table 1

| | Example 1 A | Example 2 B | Example 3 C | Example 4 D | Example 5 E | Example 6 F |
|---|---|---|---|---|---|---|
| Degree of polymerization of microcrystalline cellulose | 153 | 153 | 153 | 153 | 245 | 135 |
| Size of particle in cellulose dispersion [$\mu$m] | 7 | 7 | 7 | 7 | 9 | 5 |
| pH of cellulose dispersion | 7.0 | 7.1 | 6.9 | 7.2 | 6.8 | 7.3 |
| IC of cellulose dispersion [$\mu$S/cm] | 61 | 60 | 65 | 67 | 58 | 63 |
| Loss on drying [%] | 3.4 | 3.2 | 3.9 | 3.0 | 3.3 | 3.6 |
| Aspect ratio | 0.85 | 0.85 | 0.87 | 0.81 | 0.78 | 0.89 |
| Tapped bulk density f 9/ro1-1 | 0.78 | 0.77 | 0.80 | 0.74 | 0.75 | 0.83 |
| Angle of repose [°] | 32 | 32 | 36 | 31 | 36 | 30 |
| Shape factor | 1.28 | 1.30 | 1.26 | 1.32 | 1.29 | 1.22 |
| Average particle size [$\mu$m] | 93 | 99 | 55 | 161 | 235 | 85 |
| Loading peak value [mN] | 363 | 399 | 258 | 453 | 312 | 320 |
| Specific surface area [m2/g] | 0.26 | 0.26 | 0.28 | 0.25 | 0.25 | 0.27 |
| Water vapor absorption [%] | 2.54 | 2.53 | 2.73 | 2.45 | 2.39 | 2.67 |

Table 2

| | Example 7 G | Example 8 H | Example 9 I | Example 10 J | Example 11 K | Example 12 L |
|---|---|---|---|---|---|---|
| Degree of polymerization of microcrystalline cellulose | 270 | 270 | 153 | 153 | 153 | 153 |
| Size of particle in cellulose dispersion [$\mu$m] | 12 | 14 | 7 | 7 | 7 | 7 |
| pH of cellulose dispersion | 6.5 | 6.4 | 6.9 | 7.0 | 6.8 | 7.0 |
| IC of cellulose dispersion [$\mu$S/cm] | 55 | 57 | 66 | 64 | 60 | 68 |
| Loss on drying [%] | 3.5 | 3.4 | 3.8 | 3.8 | 3.5 | 2.9 |
| Aspect ratio | 0.72 | 0.77 | 0.83 | 0.85 | 0.83 | 0.80 |

Table continued

| | Example 7 G | Example 8 H | Example 9 I | Example 10 J | Example 11 K | Example 12 L |
|---|---|---|---|---|---|---|
| Tapped bulk density [g/ml] | 0.68 | 0.64 | 0.79 | 0.80 | 0.77 | 0.73 |
| Angle of repose [°] | 38 | 37 | 31 | 33 | 34 | 31 |
| Shape factor | 1.40 | 1.42 | 1.25 | 1.21 | 1.46 | 1.36 |
| Average particle size [$\mu$m] | 180 | 372 | 110 | 95 | 103 | 223 |
| Loading peak value [mN] | 169 | 193 | 334 | 230 | 205 | 583 |
| Specific surface area [$m^2$/g] | 0.20 | 0.19 | 0.37 | 0.53 | 0.58 | 0.37 |
| Water vapor absorption [%] | 1.95 | 1.72 | 2.50 | 2.50 | 2.42 | 2.39 |

Table 3

| | Example 13 M | Example 14 N | Example 15 O | Comparative Example 1 P | Comparative Example 2 Q | Comparative Example 3 R |
|---|---|---|---|---|---|---|
| Degree of polymerization of microcrystalline cellulose | 153 | 135 | 153 | 153 | 400 | 400 |
| Size of particle in cellulose dispersion [$\mu$m] | 6 | 5 | 7 | 17 | 25 | 13 |
| pH of cellulose dispersion | 7.3 | 7.1 | 7.1 | 7.1 | 7.5 | 7.4 |
| IC of cellulose dispersion [$\mu$S/cm) | 65 | 64 | 66 | 67 | 71 | 69 |
| Loss on drying, [%] | 3.4 | 3.8 | 3.6 | 3.4 | 3.2 | 3.8 |
| Aspect ratio | 0.78 | 0.84 | 0.82 | 0.83 | 0.65 | 0.71 |
| Tapped bulk density [g/ml] | 0.79 | 0.90 | 0.75 | 0.52 | 0.30 | 0.45 |
| Angle of repose [°] | 40 | 34 | 36 | 39 | 55 | 46 |
| Shape factor | 1.11 | 1.28 | 1.36 | 1.46 | 1.59 | 1.55 |
| Average particle size [$\mu$m] | 25 | 123 | 107 | 128 | 222 | 165 |
| leading peak value [mN] | <50 | 465 | 172 | 111 | 95 | 125 |
| Specific surface area [$m^2$/g] | 0.30 | 0.28 | 0.49 | 0.69 | 0.75 | 0.62 |

Table continued

| | Example 13 M | Example 14 N | Example 15 O | Comparative Example 1 P | Comparative Example 2 Q | Comparative Example 3 R |
|---|---|---|---|---|---|---|
| Water vapor absorption [%] | 2.18 | 2.41 | 2.21 | 2.86 | 0.73 | 2.56 |

Table 4

| | Comparative Example 4 S | Comparative Example 5 T | Comparative Example 6 U | Comparative Example 7 V | Comparative Example 8 W | Comparative Example 9 X |
|---|---|---|---|---|---|---|
| Degree of polymerization of microcrystalline cellulose | 240 | 240 | 240 | 242 | 55 | - |
| Size of particle in cellulose dispersion [$\mu$m] | - | - | - | - | 3 | - |
| pH of cellulose dispersion | - | - | - | - | 7.5 | - |
| IC of cellulose dispersion [$\mu$S/cm] | - | - | - | - | 74 | - |
| Loss on drying [%] | 2.2 | 2.3 | 2.4 | 4.2 | 3.1 | 2.7 |
| Aspect ratio | 0.90 | 0.90 | 0.91 | 0.75 | 0.86 | 0.91 |
| Tapped bulk density [g/ml] | 0.88 | 0.89 | 0.93 | 0.41 | 0.74 | 0.78 |
| Angle of repose [°] | 28 | 27 | 27 | 41 | 39 | 32 |
| Shape factor | 1.07 | 1.07 | 1.08 | 1.53 | 1.35 | 1.51 |
| Average particle size [$\mu$m] | 136 | 165 | 245 | 153 | 59 | 378 |
| Loading peak value [mN] | 667 | 724 | 1520 | 80 | 88 | 121 |
| Specific surface area [m$^2$/g] | 0.10 | 0.10 | 0.10 | 1.07 | 0.83 | 0.38 |
| Water vapor absorption [%] | 0.87 | 1.84 | 0.60 | 2.76 | 1.89 | 1.31 |

Table 5

| | Example 16 | Example 17 | Comparative Example 10 | Comparative Example 11 |
|---|---|---|---|---|
| (Seed) particles | A | B | P | S |
| Flowability | Good | Good | Good | Good |
| Friability of particles | Little | Little | Large | Large |
| Adhesion to bag filters | Little | Little | Many | Many |

Table 6

|  | Example 18 | Comparative Example 12 |
|---|---|---|
| (Seed) particles | H | X |
| Flowability | Good | Good |
| Friability of particles | Little | Large |
| Adhesion to bag filters | Little | Many |

Table 7

|  | Example 19 | Comparative Example 13 |
|---|---|---|
| (Seed) particles | C | W |
| Flowability | Good | Good |
| Friability of particles | Little | Large |
| Adhesion to bag filters | Little | Many |

Table 8

|  | Example 20 | Comparative Example 14 |
|---|---|---|
| (Seed) particles | M | P(38 $\mu$m pass) |
| Flowability | Good | Relatively good |
| Friability of particles | Little | Large |
| Adhesion to bag filters | Little | Many |

Table 9

|  | Example 21 | Comparative Example 15 | Comparative Example 16 |
|---|---|---|---|
| (Seed) particles | A | S | V |
| Definition of coarse particles | 177 $\mu$m on | 177 $\mu$m on | 177 $\mu$m on |
| Occurrence of coarse particles (%) | 1.1 | 9.4 | 18.6 |

Table 10

|  | Example 22 | Comparative Example 17 |
|---|---|---|
| (Seed) particles | B | T |
| Definition of coarse particles | 212 $\mu$m on | 212 $\mu$m on |
| Occurrence of coarse particles (%) | 1.0 1.0 | 7.8 7.8 |

Table 11

|  | Example 23 | Comparative Example 18 |
|---|---|---|
| (Seed) particles | A | S |
| Definition of coarse particles | 212 $\mu$m on | 212 $\mu$m on |

Table continued

|  | Example 23 | Comparative Example 18 |
|---|---|---|
| Occurrence of coarse particles (%) | 8.4 | 21.0 |

Table 12

|  | Example 24 | Comparative Example 19 |
|---|---|---|
| (Seed) particles | B | T |
| Definition of coarse particles | 250 $\mu$m on | 250 $\mu$m on |
| Occurrence of coarse particles (%) | 5.3 | 16.3 |

Table 13

|  | Example 25 | Comparative Example 20 |
|---|---|---|
| (Seed) particles | G (212 $\mu$m on) | T |
| Definition of coarse particles | 350 $\mu$m on | 350 $\mu$m on |
| Occurrence of coarse particles (%) | 3.0 | 10.3 |

Table 14

|  | Example 26 | Comparative Example 21 |
|---|---|---|
| (Seed) particles | M | P(38 $\mu$m pass) |
| Definition of coarse particles | 38 $\mu$m on | 38 $\mu$m on |
| Occurrence of coarse particles | 13.0 | 32.2 |

Table 15

|  | Example 27 | Comparative Example 20 |
|---|---|---|
| Preparation of granules | Example 21 | Comparative Example 16 |
| Definition of coarse particles | 212 $\mu$m on | 212 $\mu$m on |
| Occurrence of coarse particles (%) | 3.5 | 21.6 |
| Sensory analysis | No bitterness | Bitterness |

INDUSTRIAL APPLICATION

The cellulose particles for pharmaceuticals in accordance with the present invention have a proper tapped bulk density, a proper shape factor, a high aspect ratio, a proper water absorption and a proper particle strength, and are therefore extremely well suited as particles used for pharmaceuticals, particularly as seed particles to be coated with active ingredients.

**Claims**

1.  Cellulose particles for pharmaceuticals, which contain microcrystalline cellulose having an average degree of polymerization of from 60 to 350 in an amount of not less than 10%, and which have a tapped bulk density of from 0.60 to 0.95 g/ml, an aspect ratio of not less than 0.7, a shape factor of from 1.10 to 1.50, and an average particle size of from 10 to 400 $\mu$m.

**2.** The cellulose particles for pharmaceuticals according to Claim 1, wherein the particles have a specific surface area of from 0.15 to 0.60 m$^2$/g.

**3.** The cellulose particles for pharmaceuticals according to Claim 1 or 2, wherein the particles have a water vapor absorption of not less than 1.50%.

**4.** The cellulose particles for pharmaceuticals according to any one of Claims 1 to 3, wherein the particles have a loading peak value of from 130 to 630 mN.

**5.** The cellulose particles for pharmaceuticals according to any one of Claims 1 to 4, wherein the average particle size is from 40 to 400 $\mu$m.

**6.** The cellulose particles for pharmaceuticals according to any one of Claims 1 to 5, wherein the shape factor is from 1.15 to 1.50.

**7.** The cellulose particles for pharmaceuticals according to any one of Claims 1 to 6, wherein the tapped bulk density is from 0.60 to 0.90 g/ml.

**8.** The cellulose particles for pharmaceuticals according to any one of Claims 1 to 7, wherein the tapped bulk density is from 0.60 to 0.85 g/ml.

**9.** A process for producing the cellulose particles for pharmaceuticals according to any one of Claims 1 to 8, which comprises the steps of hydrolyzing a cellulose material so as to obtain an average degree of polymerization of from 60 to 350, successively milling a thus hydrolyzed product so as to obtain an average particle size of not more than 15 $\mu$m, preparing a dispersion containing the obtained microcrystalline cellulose, forming said dispersion into droplets, and thereafter drying said droplets.

**10.** The process according to Claim 9, wherein the dispersion containing the microcrystalline cellulose, which has a solids content of not less than 1%, is formed into droplets using a rotary disk at a rotating speed of from 500 to 30000 rpm, followed by drying.

**11.** Spherical granules containing the cellulose particles for pharmaceuticals according to any one of Claims 1 to 8 as seed particles, wherein an active ingredient is contained on the surface of or inside the cellulose particles.

**Patentansprüche**

**1.** Celluloseteilchen für Pharmaka, die mikrokristalline Cellulose mit einem mittleren Polymerisationsgrad von 60 bis 350 in einer Menge von nicht weniger als 10% enthalten und die eine gerüttelte Schüttdichte von 0,60 bis 0,95 g/ml, ein Aspektverhältnis von nicht weniger als 0,7, einen Formfaktor von 1,10 bis 1,50 und eine mittlere Teilchengröße von 10 bis 400 $\mu$m haben.

**2.** Celluloseteilchen für Pharmaka gemäß Anspruch 1, wobei die Teilchen eine spezifische Oberfläche von 0,15 bis 0,60 m$^2$/g haben.

**3.** Celluloseteilchen für Pharmaka gemäß Anspruch 1 oder 2, wobei die Teilchen eine Wasserdampfabsorption von nicht weniger als 1,50% haben.

**4.** Celluloseteilchen für Pharmaka gemäß einem der Ansprüche 1 bis 3, wobei die Teilchen einen Belastungsspitzenwert von 130 bis 630 mN haben.

**5.** Celluloseteilchen für Pharmaka gemäß einem der Ansprüche 1 bis 4, wobei die mittlere Teilchengröße 40 bis 400 $\mu$m beträgt.

**6.** Celluloseteilchen für Pharmaka gemäß einem der Ansprüche 1 bis 5, wobei der Formfaktor 1,15 bis 1,50 beträgt.

**7.** Celluloseteilchen für Pharmaka gemäß einem der Ansprüche 1 bis 6, wobei die gerüttelte Schüttdichte 0,60 bis 0,90 g/ml beträgt.

**8.** Celluloseteilchen für Pharmaka gemäß einem der Ansprüche 1 bis 7, wobei die gerüttelte Schüttdichte 0,60 bis 0,85 g/ml beträgt.

**9.** Verfahren zur Herstellung der Celluloseteilchen für Pharmaka gemäß einem der Ansprüche 1 bis 8, das die folgenden Schritte umfasst: Hydrolysieren eines Cellulosematerials, so dass man einen mittleren Polymerisationsgrad von 60 bis 350 erhält, sukzessives Mahlen des so hydrolysierten Produkts, so dass man eine mittlere Teilchengröße von nicht mehr als 15 $\mu$m erhält, Herstellen einer Dispersion, die die erhaltene mikrokristalline Cellulose enthält, Bilden von Tröpfchen aus der Dispersion und danach Trocknen der Tröpfchen.

**10.** Verfahren gemäß Anspruch 9, wobei aus der Dispersion, die die mikrokristalline Cellulose enthält und die einen Feststoffgehalt von nicht weniger als 1% hat, Tröpfchen unter Verwendung eines Drehtellers mit einer Rotationsgeschwindigkeit von 500 bis 30 000 U/min gebildet und anschließend getrocknet werden.

**11.** Sphärisches Granulat, das die Celluloseteilchen für Pharmaka gemäß einem der Ansprüche 1 bis 8 als Starterkerne enthält, wobei auf der Oberfläche oder innerhalb der Celluloseteilchen ein Wirkstoff enthalten ist.


**Revendications**

**1.** Particules de cellulose pour produits pharmaceutiques, qui contiennent de la cellulose microcristalline ayant un degré moyen de polymérisation de 60 à 350 en une quantité non inférieure à 10 %, et qui ont une masse volumique apparente tassée de 0,60 à 0,95 g/ml, un indice de forme non inférieur à 0,7, un facteur de forme de 1,10 à 1,50 et une taille moyenne de particules de 10 à 400 $\mu$m.

**2.** Particules de cellulose pour produits pharmaceutiques selon la revendication 1 où les particules ont une surface spécifique de 0,15 à 0,60 m$^2$/g.

**3.** Particules de cellulose pour produits pharmaceutiques selon la revendication 1 ou 2 où les particules ont une absorption de vapeur d'eau non inférieure à 1,50 %.

**4.** Particules de cellulose pour produits pharmaceutiques selon l'une quelconque des revendications 1 à 3 où les particules ont une valeur maximale de charge de 130 à 630 mN.

**5.** Particules de cellulose pour produits pharmaceutiques selon l'une quelconque des revendications 1 à 4 où la taille moyenne de particules est de 40 à 400 $\mu$m.

**6.** Particules de cellulose pour produits pharmaceutiques selon l'une quelconque des revendications 1 à 5 où le facteur de forme est de 1,15 à 1,50.

**7.** Particules de cellulose pour produits pharmaceutiques selon l'une quelconque des revendications 1 à 6 où la masse volumique apparente tassée est de 0,60 à 0,90 g/ml.

**8.** Particules de cellulose pour produits pharmaceutiques selon l'une quelconque des revendications 1 à 7 où la masse volumique apparente tassée est de 0,60 à 0,85 g/ml.

**9.** Procédé pour produire les particules de cellulose pour produits pharmaceutiques selon l'une quelconque des revendications 1 à 8 qui comprend les étapes d'hydrolyse d'une matière cellulosique de manière à obtenir un degré moyen de polymérisation de 60 à 350, puis de broyage d'un produit ainsi hydrolysé de manière à obtenir une taille moyenne de particules non supérieure à 15 $\mu$m, de préparation d'une dispersion contenant la cellulose microcristalline obtenue, de transformation de ladite dispersion en gouttelettes, puis de séchage desdites gouttelettes.

**10.** Procédé selon la revendication 9 où la dispersion contenant la cellulose microcristalline, qui a une teneur en solides non inférieure à 1 %, est transformée en gouttelettes au moyen d'un disque rotatif à une vitesse de rotation de 500 à 30 000 tr/min, après quoi elle est séchée.

**11.** Granules sphériques contenant les particules de cellulose pour produits pharmaceutiques selon l'une quelconque des revendications 1 à 8 sous forme de particules d'ensemencement, où un ingrédient actif est contenu sur la surface ou à l'intérieur des particules de cellulose.